# EUROPEAN PATENT APPLICATION

(11) **EP 4 498 081 A1**
(43) Date of publication of application: **29.01.2025**
(21) Application number: 24184331.7
(22) Date of filing: 25.06.2024
(51) Int. Cl.: G01N 33/00, G01N 21/78, G01N 31/22

(54) **METHODS AND APPARATUSES FOR DETERMINING CONCENTRATION OF A TARGET GAS WITHIN A SAMPLE GAS**

(30) Priority: 27.07.2023 CN 202310937361
(71) Applicant: Honeywell International Inc., Charlotte, NC 28202 (US)
(72) Inventor: YU, Yan, Charlotte, 28202 (US); HUANG, Chuang, Charlotte, 28202 (US); HOU, Zhixiong, Charlotte, 28202 (US); LIU, Jiangtao, Charlotte, 28202 (US); HE, Xiang, Charlotte, 28202 (US); HUANG, Yongfang, Charlotte, 28202 (US); ZHAO, Linan, Charlotte, 28202 (US)
(74) Representative: Haseltine Lake Kempner LLP

(57) **Abstract**

Methods and apparatusesfor determining a concentration of a target gas in a sample gas are provided. An example method of preparing a replaceable testing material for detecting a target gas in a sample gas may include preparing a solution comprising a solute and a solvent, wherein the solute comprises o-tolidine; introducing a testing material substrate to the prepared solution for a period of time; and removing at least a portion of the solvent from the testing material substrate such that the testing material substrate comprises at least a portion of the o-tolidine, and thereby forming the replaceable testing material for detecting the target gas in the sample gas.

## Description

### TECHNOLOGICAL FIELD

Embodiments of the present disclosure relate generally to determining the concentration of a target gas within a sample gas, and more particularly, to determining the concentration of nitric oxide (NO) within a sample gas using a replaceable testing material in a care setting or medical environment.

### BACKGROUND

Applicant has identified a number of deficiencies and technical problems associated with conventional systems, apparatuses, and methods for determining the concentration of a target gas within a sample gas, particularly in a medical environment. Through applied effort, ingenuity, and innovation, many of these identified problems have been solved by developing solutions that are included in embodiments of the present disclosure, many examples of which are described in detail herein.

### BRIEF SUMMARY

In general, various embodiments of the present disclosure provided herein may provide solutions to the problems and needs in the art that have not yet been fully identified, appreciated, or solved by current technologies for determining the concentration of a target gas within a sample gas. In accordance with some embodiments of the present disclosure, an example method of preparing a replaceable testing material for determining a concentration of a target gas in a sample gas is provided. In some embodiments, the method may include preparing a solution comprising a solute and a solvent, wherein the solute comprises *o*-tolidine; introducing a testing material substrate to the prepared solution for a period of time; and removing at least a portion of the solvent from the testing material substrate such that the testing material substrate comprises at least a portion of the *o*-tolidine, and thereby forming the replaceable testing material for determining a concentration of target gas in the sample gas.

In some embodiments, removing at least the portion of the solvent from the testing material substrate may include evaporating the solvent (e.g., ethanol). In some embodiments, removing at least the portion of the solvent from the testing material substrate may include applying absorption paper to the testing material substrate. In some further embodiments, removing at least the portion of the solvent from the testing material substrate may include exposing the testing material substrate to a nitrogen gas flow.

In some embodiments, the solvent may include ethanol. In some further embodiments, the solvent may include water.

In some embodiments, the period of time for introducing the testing material substrate to the prepared solution may be a minimum of about 30 seconds.

In some embodiments, the testing material substrate comprises low speed test paper.

In accordance with some embodiments of the present disclosure, an example method for determining a concentration of a target gas in a sample gas is further provided. In some embodiments, the method may include contacting a replaceable testing material with the sample gas, wherein at least a portion of the sample gas is the target gas and wherein the replaceable testing material comprises *o*-tolidine; identifying a color of the replaceable testing material; and determining the concentration of the target gas in the sample gas based at least in part on the identified color of the replaceable testing material.

In some embodiments, determining the concentration of the target gas in the sample gas may include determining a concentration of an oxidized derivative of the target gas and correlating the determined concentration of the oxidized derivative to the concentration of the target gas. In some further embodiments, the target gas is nitric oxide and the oxidized derivative is nitrogen dioxide.

In some embodiments, contacting the replaceable testing material with the sample gas may include exposing the replaceable testing material to a sample gas flow. In some further embodiments, the replaceable testing material is disposed in a gas detection apparatus and exposing the replaceable testing material to the sample gas flow may include directing a sample gas flow toward the replaceable testing material in the gas detection apparatus. In some further embodiments, the sample gas may include the target gas and after contacting the replaceable testing material with the sample gas, the replaceable testing material changes color based at least in part on a presence of the target gas. In still further embodiments, the identified color of the replaceable testing material may be blue, green, or a combination thereof.

In accordance with some embodiments of the present disclosure, an example reusable gas detection apparatus for determining a concentration of a target gas in a sample gas is provided. In some embodiments, the example reusable gas detection apparatus may include a replaceable testing material configured to change color based at least in part on a presence of a target gas in the sample gas or an oxidized derivative of the target gas in the sample gas, wherein the replaceable testing material comprises *o*-tolidine; and a controller configured to determine the concentration of the target gas.

In some embodiments, the reusable gas detection apparatus may further include an imaging device and the controller may be configured to determine the concentration of the target gas based at least in part on correlating the color of the replaceable testing material in one or more images of the replaceable testing material captured by the imaging device to the concentration of the target gas.

In some embodiments, the controller may be configured to determine the concentration of the target gas based at least in part on determining a concentration of the oxidized derivative of the target gas.

In some embodiments, the reusable gas detection apparatus may include an oxidization chamber comprising an oxidizing agent, wherein the oxidizing agent oxidizes at least a portion of the target gas in the sample gas to the oxidized derivative of the target gas prior to the sample gas reaching the replaceable testing material. In some further embodiments, the target gas is nitric oxide and the oxidized derivative is nitrogen dioxide. In still further embodiments, the reusable gas detection apparatus may include an integrated display configured to present a result of the determined concentration of the nitric oxide in the sample gas.

### BRIEF DESCRIPTION OF THE DRAWINGS

Having thus described the present disclosure in general terms, reference will now be made to the accompanying drawings, which are not necessarily drawn to scale. The components illustrated in the figures may or may not be present in certain embodiments described herein. Some embodiments may include fewer (or more) components than those shown in the figures in accordance with an example embodiment of the present disclosure.
FIG. 1 illustrates an exploded view of an example gas detection apparatus in accordance with some example embodiments described herein.
FIG. 2 illustrates a schematic block diagram showing example components of a color meter reader in accordance with some example embodiments described herein.
FIG. 3 illustrates a schematic block diagram showing example components of a controller in accordance with some example embodiments described herein.
FIG. 4 depicts a flowchart illustrating an example method of preparing a replaceable testing material for determining a concentration of a target gas within a sample gas in accordance with some example embodiments described herein.
FIG. 5 depicts a flowchart illustrating an example method for determining a concentration of a target gas in a sample gas in accordance with some example embodiments described herein.

### DETAILED DESCRIPTION

Example embodiments of the present disclosure will now be described more fully hereinafter with reference to the accompanying drawings, in which some, but not all embodiments of the disclosure are shown. Indeed, embodiments of the disclosure may be embodied in many different forms and should not be construed as limited to the embodiments set forth herein; rather, these embodiments are provided so that this disclosure will satisfy applicable legal requirements. Like numbers refer to like elements throughout.

Various example embodiments address technical problems associated with determining the concentration of a target gas contained within a sample gas. As understood by those of skill in the field to which the present disclosure pertains, there are numerous example scenarios in which a user may seek to determine the concentration of a target gas within a sample gas. For example, there are numerous environmental- and health-related contexts in which the determination of the concentration of a target gas, such as nitric oxide (NO), in a sample gas may be desired or necessary. However, due to rapid chemical reactions with a wide range of molecules, some target gases, such as nitric oxide, may be particularly difficult to measure, especially in medical contexts.

The use of nitric oxide in various medical treatments has become widespread. The presence of nitric oxide in biological materials has been linked with various beneficial effects within health contexts, particularly due to the effect nitric oxide has at a cellular level. For example, nitric oxide has been utilized in wound healing therapy to realize improvements in inflammatory response to damaged cells, cell growth and regrowth, new blood vessel formation through collagen formation, and preventing the formation of microbrial colonies. Nitric oxide has also been proven to show improvements in anticancer activity within the body. In addition, nitric oxide has been utilized in respiratory applications and cardiovascular applications showing improvements in the operations of these bodily systems. Significant research continues to be performed into the development and assessment of nitric oxide donors, such as dendrimers, metal-organic frameworks (MOFs), micelles, inorganic nanoparticles (NPs), and gels, used to deliver nitric oxide to the various biological systems that may benefit from an increase in nitric oxide. Due to the numerous physiological and pathological benefits of nitric oxide, regulation of nitric oxide levels in various tissues may have significant therapeutic value. Further, the regulation of nitric oxide levels may necessarily include the accurate measurement of nitric oxide in various sample gases.

Many example methods for measuring nitric oxide are either costly, inaccurate, unreliable, not suited for medical or point of care applications, and/or difficult to administer. Chemiluminescence, for example, utilizes the chemiluminescent nature of nitric oxide when reacted with ozone to determine the amount of nitric oxide in a particular substance based on the light output. Chemiluminescence may be generally used in a laboratory and/or hospital environment, however, the expense of chemiluminescence may be prohibitively high, such that use of chemiluminescence techniques may be limited to research contexts, such as in large-scale hospitals and universities. In addition, the detection time is long and the process is complicated, such that a highly trained operator may be necessary to perform the tests. Further, the detection system must be periodically calibrated to obtain accurate results.

Gas chromatography may use analytical chemistry for separating and analyzing compounds that can be vaporized without decomposition. Gas chromatography may be better suited for laboratory, environmental, and/or industrial applications and is not easily performed for an individual or patient in a health care environment. In addition, gas chromatography may be prohibitively expensive and, similar to chemiluminescence, detection time is long and the process is complicated, such that a highly trained operator may be necessary to perform the tests. Further, the detection system must be periodically calibrated to obtain accurate results.

Electrochemical sensors may produce an electric current from energy released by a spontaneous redox reaction. Electrochemical sensors, however, may be difficult to calibrate and operate, such that a highly trained operator may be necessary to administer the test process. In addition, the expense of detecting nitric oxide using electrochemical sensor may be significant.

Spectral absorption may use spectroscopic techniques to measure the absorption of radiation as a function of the frequency of wavelength of incident light. Due to the interaction of the incident light with the sample gas, the concentration of nitric oxide may be detected. However, spectral absorption may be better suited for laboratory and/or environment applications and not easily performed for an individual or patient in a health care environment. In addition, the test duration of spectral absorption may be long. Further, the cost per test to perform spectral absorption may be prohibitively expensive and the test performance process is complicated, such that a highly trained operator may be necessary to perform the tests and periodically calibrate the test equipment.

Various embodiments of the present disclosure provide methods and apparatuses to determine the concentration of a target gas such as nitric oxide in a sample gas. The inventors have determined it would be desirable and advantageous to be able to more easily and efficiently determine the concentration of nitric oxide in a sample gas. As a result of the herein described example embodiments and in some examples, the effectiveness and ease of use of a gas detection apparatus capable of detecting nitric oxide may be greatly improved. For example, in some embodiments, although the gas detection apparatus may be reusable, the measurement component (e.g., replaceable testing material) of the gas detection apparatus may be disposable (i.e., replaceable) such that the testing material is used only once, reducing the possibility of exposing a patient to any possible contamination from a previous patient. Moreover, the cost per test of a sample gas may be low as a result of the replaceable testing material and the replaceable testing material may not require periodic calibration for accurate operation due to prior use. These characteristics as well as additional features, functions, and details are described below. Similarly, corresponding and additional embodiments are also described below.

### Example Apparatuses of the Disclosure

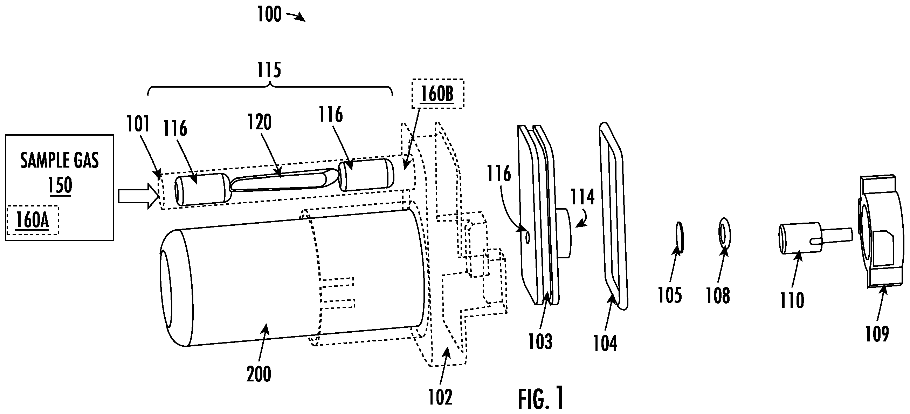

The replaceable testing material 105 of the present disclosure may be used in various gas detection apparatuses to and is not limited to the gas detection apparatuses described herein. Referring now to FIG. 1, an example gas detection apparatus 100 according to one example embodiment is illustrated. As depicted in FIG. 1, the example gas detection apparatus 100 may be configured to receive a sample gas 150, for example, via a gas inlet 101. The gas inlet 101 may be any hose, tube, nozzle, or similar structure configured to receive the sample gas 150 from a gas source. A sample gas 150 may be any gas, fluid, and/or substance received from any such gas source. For example, the sample gas 150 may be received from a subject, such as a human subject, environment, machine, cavity, space, or other biological or environmental source. The source may be direct (e.g., by a subject exhaling directly into the gas inlet 101) or indirect (e.g., from a sample bag). The sample gas 150 may be comprised of a plurality of gases and molecules.

In some embodiments, the sample gas 150 may potentially include a target gas 160A. A target gas 160A may comprise any gas, fluid, and/or substance from any source. A target gas 160A may be one or more gases for which the gas detector apparatus 100 is configured to detect. For example, in some embodiments, a target gas 160A may be nitric oxide (NO). A target gas 160A may comprise a gas concentration within the sample gas 150. A gas concentration refers to the amount of gas (e.g., mass, atoms, molecules, moles) present in a certain volume at a certain time. A gas concentration for a target gas 160A may be measured as a ratio of the number of particles of the target gas 160A to the total number of particles in the sample gas 150. In some embodiments, the concentration measurement may be expressed as a ratio such as parts per million (ppm) or parts per billion (ppb). In some embodiments, the replaceable testing material 105 may be used to detect the concentration of a target gas 160A and/or oxidized derivative 160B of the target gas as low as 5 ppb.

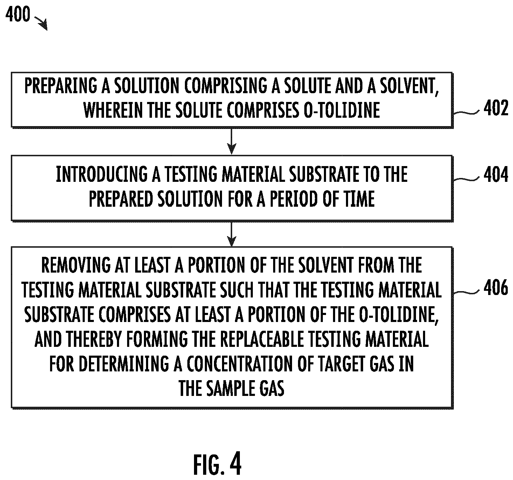

As depicted in FIG. 1, the example gas detection apparatus 100 may include a replaceable testing material 105. Although the gas detection apparatus 100 itself may be reusable, the replaceable testing material 105 is configured to be a consumable (e.g., one-time use, disposable, replaceable component). As depicted in FIG. 1, the replaceable testing material 105 may be disposed within the gas detection apparatus 100, for example, within a flow of the sample gas 150 (e.g., sample gas flow). The replaceable testing material 105 may comprise any material configured to react with a stimuli (e.g., a target gas 160A or oxidized derivative 160B thereof), such that when the replaceable testing material 105 is exposed to the stimuli, the replaceable testing material 105 changes color. In such embodiments, the replaceable testing material 105 is a colorimetric indicator of the presence of the stimuli (e.g., nitric oxide or any oxidized derivative, such as nitrogen dioxide (NO₂)). In other words, in some embodiments, the color and/or shade of the replaceable testing material 105 may be an indicator of the concentration of the target gas 160A (e.g., or its oxidized derivative 160B) within the sample gas 150. For example, in some embodiments, the darker the shade of the color, the higher the concentration of the target gas 160A or its oxidized derivative 160B. For example, in some embodiments, different shades of blue, green, and/or blue-green may indicate different concentrations of the target gas 160A or its oxidized derivative 160B. Or, for example, in some embodiments, yellow may indicate a low concentration and red may indicate a high concentration, with different colors based on the various concentration ranges. Positioning the replaceable testing material 105 near or withing a sample gas flow may provide more accurate measurements of the concentration of the target gas 160A or its oxidized derivative 160B within the sample gas 150. Example methods of preparing the replaceable testing material 105 for determining a concentration of a target gas 160A in a sample gas 150 are further described in relation to FIG. 4.

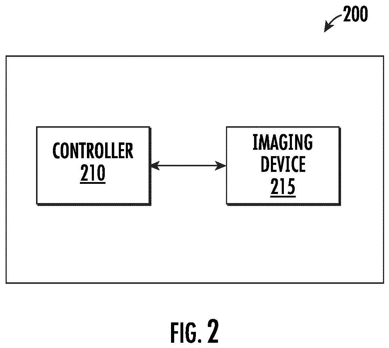

As further depicted in FIG. 1, the example gas detection apparatus 100 may include a color meter reader 200. The color meter reader 200 may be any device comprising sensing elements and compute components configured to determine the concentration of the target gas 160A and/or oxidized derivative 160B of the target gas within the sample gas 150 based on measurements derived from the replaceable testing material 105. For example, an example block diagram of an example color meter reader 200 is provided in FIG. 2. As depicted in FIG. 2, a color meter reader 200 may comprise an imaging device 215. An imaging device 215 may be any device, module, or other apparatus configured to capture imagery data (e.g., one or more images of the replaceable testing material 105). An imaging device 215 may comprise a lens, an image sensor (e.g., charge-coupled device sensor, complementary metal-oxide-semiconductor sensor), and/or communication circuitry. In some embodiments, the imaging device 215 may be directed to capture imagery data of the replaceable testing material 105. In some embodiments, the image sensor may be configured to capture color imagery, enabling the gas detection apparatus 100 to determine the concentration of the target gas 160A and/or oxidized derivative 160B of the target gas within the sample gas 150 based at least in part on the color of the replaceable testing material 105. In some embodiments, the imaging device 215 may comprise an on-board processor, supporting, among other things, image processing functions.

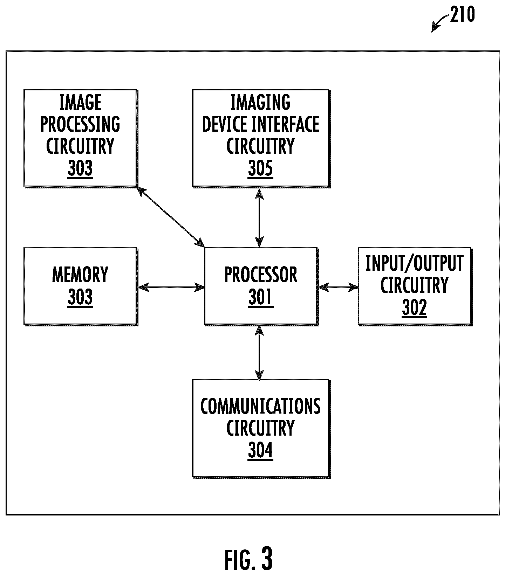

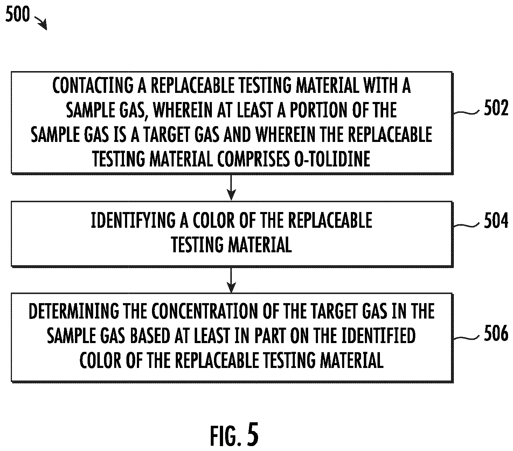

As depicted in FIG. 2, the imaging device 215 may be electrically and communicatively connected to a controller 210, enabling the imaging device 215 to transmit captured imagery data to the controller 210 for further processing. The change in color of the replaceable testing material 105 may be monitored and/or identified by the controller 210, as further described in relation to FIG. 5. For example, based at least in part on the images received from the imaging device 215, the controller 210 of the color meter reader 200 may determine the concentration of the target gas 160A and/or oxidized derivative 160B of the target gas within the sample gas 150. An example controller 210 is further described in conjunction with FIG. 3.

Returning to FIG. 1, the example gas detection apparatus 100 may include an oxidization chamber 115. In some embodiments, as depicted in FIG. 1, the oxidization chamber 115 may be disposed within the gas inlet 101 of the gas detection apparatus 100. The oxidization chamber 115 may include one or more gas filters 116. A gas filter 116 may comprise any material, structure, or device or combination of materials, structures or devices configured to allow a sample gas 150 to flow through while substantially removing volatile organic compounds (VOCs) molecules and/or moisture contained within the sample gas 150. Non-limiting examples of a gas filter 116 may include cellulose acetate, polypropylene, paper, cotton, charcoal, cork, and other permeable materials. In some embodiments, a combination of materials may be utilized as a gas filter 116, for example, a first material may be utilized to remove VOCs and a second material may be utilized to remove moisture from the sample gas 150.

As further depicted in FIG. 1, the example oxidization chamber 115 of the gas detection apparatus 100 may include an oxidizing agent 120. For example, as depicted in FIG. 1, a first gas filter 116 may be disposed near a proximate end of the oxidization chamber 115 and a second gas filter 116 may be disposed near a terminal end of the oxidization chamber 115 and the oxidizing agent 120 may be disposed therebetween. An oxidizing agent 120 may be any chemical, compound, material, or other substance that may readily transfer an oxygen atom to the target gas 160A in the sample gas 150. That is, the oxidizing agent 120 oxidizes at least a portion of the target gas 160A in the sample gas 150 to the oxidized derivative 160B of the target gas prior to the sample gas 150 reaching the replaceable testing material 105 disposed in the gas detection apparatus 100. For example, in an instance in which nitric oxide is the target gas 160A, an oxidizing agent 120, such as metallic oxide compounds or potassium permanganate (KMnO₄), may be used. The oxidizing agent may transfer an oxygen atom to the nitric oxide molecules as they pass through the oxidization chamber 115. In some embodiments, such oxidation results in the oxidized derivative nitrogen oxide (NO₂). Nitrogen dioxide may be more stable and less susceptible to reaction with other chemical molecules than nitric oxide. Accordingly, measuring the concentration of the resulting nitrogen dioxide may provide an easier and a more accurate representation of the amount of nitric oxide in the sample gas 150 than attempting to directly measure the concentration of nitric oxide.

As further depicted in FIG. 1, the example gas detection apparatus 100 may optionally include a housing 102 configured to receive the sample gas 150 via a gas inlet 101 and direct a flow of the sample gas 150 (e.g., sample gas flow) toward the replaceable testing material 105. The optional housing 102 may also be configured to receive and/or attach to a cover 103. A cover ring 104 (e.g., an O-ring) may surround the exterior edge of the cover 103 to create an air tight seal between the cover 103 and the walls of the housing 102. The cover 103 may include a gas outlet opening 116 and a cover opening 114, the gas outlet opening 116 arranged to receive the sample gas flow from the housing 102 and direct the sample gas flow out through the cover opening 114 disposed opposite the gas outlet opening 116. The cover opening 114 may optionally be further configured to receive additional components, such as the replaceable testing material 105, a pressure stem 110 (e.g., to regulate gas flow), a gas outlet ring 108 (e.g., an O-ring creating an air tight seal between the pressure stem 110 and the gas outlet opening 116), and a lock 109 (e.g., to apply pressure to cover 103, ensuring and air tight seal. The housing 102 may also be configured to provide a place of attachment (e.g., comprising one or more latching mechanism such as a lock, a latch, a screw, or other mechanism) for the color meter reader 200 such that the sensing devices within the color meter reader 200 may monitor the replaceable testing material 105 during operation.

Referring now to FIG. 3, an example controller 210 in accordance with at least some example embodiments of the present disclosure is disclosed. The controller 210 includes processor 301, input/output circuitry 302, memory 303, communications circuitry 304, imaging device interface circuitry 305, and image processing circuitry 306. In some embodiments, the controller 210 is configured, using one or more of the sets of circuitry 301, 302, 303, 304, 305, and/or 306, to execute and perform the operations described herein.

Although components are described with respect to functional limitations, it should be understood that the particular implementations necessarily include the use of particular computing hardware. It should also be understood that in some embodiments certain of the components described herein include similar or common hardware. For example, two sets of circuitry may both leverage use of the same processor(s), network interface(s), storage medium(s), and/or the like, to perform their associated functions, such that duplicate hardware is not required for each set of circuitry. The user of the term "circuitry" as used herein with respect to components of the apparatuses described herein should therefore be understood to include particular hardware configured to perform the functions associated with the particular circuitry as described herein.

Particularly, the term "circuitry" should be understood broadly to include hardware and, in some embodiments, software for configuring the hardware. For example, in some embodiments, "circuitry" includes processing circuitry, storage media, network interfaces, input/output devices, and/or the like. Alternatively or additionally, in some embodiments, other elements of the controller 301 provide or supplement the functionality of other particular sets of circuitry. For example, the processor 301 in some embodiments provides processing functionality to any of the sets of circuitry, the memory 303 provides storage functionality to any of the sets of circuitry, the communications circuitry 304 provides network interface functionality to any of the sets of circuitry, and/or the like.

In some embodiments, the processor 301 (and/or co-processor or any other processing circuitry assisting or otherwise associated with the processor) is/are in communication with the memory 303 via a bus for passing information among components of the controller 210. In some embodiments, for example, the memory 303 is non-transitory and may include, for example, one or more volatile and/or non-volatile memories. In other words, for example, the memory 303 in some embodiments includes or embodies an electronic storage device (e.g., a computer readable storage medium). In some embodiments, the memory 303 is configured to store information, data, content, applications, instructions, or the like, for enabling the controller 210 to carry out various functions in accordance with example embodiments of the present disclosure.

The processor 301 may be embodied in a number of different ways. For example, in some example embodiments, the processor 301 includes one or more processing devices configured to perform independently. Additionally or alternatively, in some embodiments, the processor 301 includes one or more processor(s) configured in tandem via a bus to enable independent execution of instructions, pipelining, and/or multithreading. The use of the terms "processor" and "processing circuitry" should be understood to include a single core processor, a multi-core processor, multiple processors internal to the controller 210, and/or one or more remote or "cloud" processor(s) external to the controller 210.

In an example embodiment, the processor 301 is configured to execute instructions stored in the memory 303 or otherwise accessible to the processor. Alternatively or additionally, the processor 301 in some embodiments is configured to execute hard-coded functionality. As such, whether configured by hardware or software methods, or by a combination thereof, the processor 301 represents an entity (e.g., physically embodied in circuitry) capable of performing operations according to an embodiment of the present disclosure while configured accordingly. Alternatively or additionally, as another example in some example embodiments, when the processor 301 is embodied as an executor of software instructions, the instructions specifically configure the processor 301 to perform the algorithms embodied in the specific operations described herein when such instructions are executed.

As one particular example embodiment, the processor 301 is configured to perform various operations associated with utilizing various sensors and devices to determine the concentration of a target gas 160A in a sample gas (e.g., sample gas 150). In some embodiments, the processor 301 includes hardware, software, firmware, and/or a combination thereof, that receives from an imaging device (e.g., imaging device 215), an image of a testing material (e.g., replaceable testing material 105), wherein the testing material is positioned within a sample gas flow and wherein a color of the testing material is correlated with the concentration of the target gas (e.g., target gas 160A and/or oxidized derivative 160B thereof). Additionally or alternatively, in some embodiments, the processor 301 includes hardware, software, firmware, and/or a combination thereof, that determines the concentration of the target gas 160A and/or oxidized derivative 160B thereof based at least in part on the color of the testing material (e.g., replaceable testing material 105).

In some embodiments, the controller 210 includes input/output circuitry 302 that provides output to a user and, in some embodiments, to receive an indication of a user input. In some embodiments, the input/output circuitry 302 is in communication with the processor 301 to provide such functionality. The input/output circuitry 302 may comprise one or more user interface(s) (e.g., user interface) and in some embodiments includes a display that comprises the interface(s) rendered as a web user interface, an application user interface, a user device, a backend system, or the like. The processor 301 and/or input/output circuitry 302 comprising the processor may be configured to control one or more functions of one or more user interface elements through computer program instructions (e.g., software and/or firmware) stored on a memory accessible to the processor (e.g., memory 303, and/or the like). In some embodiments, the input/output circuitry 302 includes or utilizes a user-facing application to provide input/output functionality to a client device and/or other display associated with a user.

In some embodiments, the controller 210 includes communications circuitry 304. The communications circuitry 304 includes any means such as a device or circuitry embodied in either hardware or a combination of hardware and software that is configured to receive and/or transmit data from/to a network and/or any other device, circuitry, or module in communication with the controller 210. In this regard, the communications circuitry 304 includes, for example in some embodiments, a network interface for enabling communications with a wired or wireless communications network. Additionally or alternatively in some embodiments, the communications circuitry 304 includes one or more network interface card(s), antenna(s), bus(es), switch(es), router(s), modem(s), and supporting hardware, firmware, and/or software, or any other device suitable for enabling communications via one or more communications network(s). Additionally or alternatively, the communications circuitry 304 includes circuitry for interacting with the antenna(s) and/or other hardware or software to cause transmission of signals via the antenna(s) or to handle receipt of signals received via the antenna(s). In some embodiments, the communications circuitry 304 enables transmission to and/or receipt of data from a client device in communication with the controller 210.

The imaging device interface circuitry 305 includes hardware, software, firmware, and/or a combination thereof, that supports various functionality associated with command and control of the imaging device (e.g., imaging device 215) of a color meter reader (e.g., color meter reader 200). For example, in some embodiments, the imaging device interface circuitry 305 includes hardware, software, firmware, and/or a combination thereof, that receives, decodes, and/or otherwise processes or pre-processes captured imagery data. Additionally or alternatively, in some embodiments, the imaging device interface circuitry 305 includes hardware, software, firmware, and/or a combination thereof, that stores and/or otherwise caches imagery data for subsequent use, retrieval, transmission, and/or other processing. In some embodiments, the imaging device interface circuitry 305 includes a separate processor, specially configured field programmable gate array (FPGA), or a specially programmed application specific integrated circuit (ASIC).

In an example embodiment imaging device interface circuitry 305 may further include hardware, software, firmware, and/or a combination thereof to command and control the imaging device. Various commands may be transmitted to the imaging device to configure the imaging device based on the received imagery data. For example, imaging device interface circuitry 305 may be configured to monitor and adjust the exposure time, ISO sensitivity, white balance, shutter speed, gain, frame rate, dynamic range, bit depth, and other parameters related to the quality of the imagery data.

The image processing circuitry 306 includes hardware, software, firmware, and/or a combination thereof, that supports various functionality associated with processing the received captured imagery data from an image sensor. In an example embodiment, the image processing circuitry 306 may perform image processing techniques to prepare the captured imagery data for further processing by, for example, removing optical noise, accentuating features such as edges, determining features incident to identifying objects of interest, calculating feature values incident to recognizing objects represented by the captured imagery data, and other similar techniques known to a person of ordinary skill in the art. In some embodiments, the image processing circuitry 306 may utilize image processing techniques on the processed captured image data to identify objects of interest, for example, the test paper 306 and similar objects of interest. In some embodiments, the image processing circuitry 306 may utilize further image processing techniques to identify the color of the objects of interest according to a relevant color space, for example, red-green-blue (RGB), hue-saturation-value (HSV), hue-lightness-saturation (HLS), luminance-blue projection-red projection (YUV, YCbCr), cyan-magenta-yellow (CMY). The determined color of the object of interest (e.g., replaceable testing material 105) may be compared with the properties of the object of interest to determine the concentration of the target gas (e.g., target gas 160A and/or oxidized derivative 160B thereof).

Additionally or alternatively, in some embodiments, one or more of the sets of circuitry 301-306 are combinable. Additionally or alternatively, in some embodiments, one or more of the sets of circuitry perform some or all of the functionality described associated with another component. For example, in some embodiments, one or more sets of circuitry 301-306 are combined into a single module embodied in hardware, software, firmware, and/or a combination thereof. Similarly, in some embodiments, one or more of the sets of circuitry, for example imaging device interface circuitry 305, is/are combined such that the processor 301 performs one or more of the operations described above with respect to each of these circuitry individually.

Additionally or alternatively, in some embodiments, one or more of the sets of circuitry 301-306 are combinable. Additionally or alternatively, in some embodiments, one or more of the sets of circuitry perform some or all of the functionality described associated with another component. For example, in some embodiments, one or more sets of circuitry 301-306 are combined into a single module embodied in hardware, software, firmware, and/or a combination thereof. Similarly, in some embodiments, one or more of the sets of circuitry, for example imaging device interface circuitry 305, and/or image processing circuitry 306, is/are combined such that the processor 301 performs one or more of the operations described above with respect to each of these circuitry individually.

### Example Methods of the Disclosure

Referring to FIGS. 4 and 5, example methods for preparing a testing material for determining the concentration of a target gas (e.g., nitric oxide) in a sample gas and using such a testing material to determine the concentration of a target gas (e.g., nitric oxide) in a sample gas are illustrated, respectively. That is, FIG. 4 and FIG. 5 illustrate flowcharts containing series of steps for preparing a replaceable testing material and determining the concentration of a target gas 160A (e.g., nitric oxide) in a sample gas 150, for example, with the gas detection apparatus 100 and/or replaceable testing material 105 as described above.

Referring now to FIG. 4, an example method 400 for preparing a replaceable testing material (e.g., replaceable testing material 105) for determining a concentration of a target gas in a sample gas (e.g. sample gas 150) is illustrated. At block 402, example method 400 includes preparing a solution comprising a solute and a solvent, wherein the solute comprises *o*-tolidine. In an example embodiment, *o*-tolidine, which may also be known as 3,3'-dimethylbenzidine or 4,4'-diamino-3,3'-dimethylbiphenyl, may be in powder form and dissolved into and/or mixed with ethanol and/or water. In an example embodiment, approximately 5-20 mg of *o*-tolidine may be mixed with 1-5 mL of CH₃CH₂OH, >98%. In another example embodiment, approximately 5-20 mg of *o*-tolidine may be mixed with 1-5 mL of CH₃CH₂OH, >98% and 1-5 mL of water.

With continued reference to FIG. 4, at block 404, example method 400 includes introducing a testing material substrate to the prepared solution for a period of time. For example, in some embodiments, a testing material substrate, such as low speed test paper, may be dipped into the solution prepared at block 402 for a minimum of 30 seconds. In some further embodiments, the testing material substrate may be dipped into the solution prepared at block 402 for approximately 30-120 seconds. Low speed test paper may refer to a testing material substrate having a fibrous condition that is tighter with holes having a smaller diameter compared to typical test paper (e.g., normal speed test paper). Such low speed test paper, once prepared as a replaceable testing material 105, may provide for more uniform distribution of the solute (e.g., *o*-tolidine) once dried and allow a sample gas 150 to pass through the test paper slowly, enabling a more complete reaction of the applied solute with the target gas 160A (or oxidized derivate 160B thereof), if any, present in the sample gas 150.

As further illustrated in FIG. 4, at block 406, example method 400 includes removing at least a portion of the solvent from the testing material substrate such that the testing material substrate comprises at least a portion of the *o*-tolidine, thereby forming the replaceable testing material (e.g., replaceable testing material 105) for determining a concentration of target gas (e.g., target gas 160A) in the sample gas 150. For example, removing at least the portion of the solvent from the testing material substrate may include applying absorption paper and/or wrapping the testing material substrate with absorption paper (e.g., water absorption paper). In some embodiments, the absorption paper is contacted with the testing material substrate for approximately 30-120 seconds. Additionally or alternatively, removing at least the portion of the solvent from the testing material substrate may include blowing an inert gas, such as nitrogen gas, on the testing material substrate. That is, the testing material substrate may be exposed to a nitrogen gas flow for a period of time, such as approximately 30-120 seconds. The prepared replaceable testing material 105 may be stored in shaded packaging at approximately -20°C to below 4°C. Additionally or alternatively, the prepared replaceable testing material 105 may be sealed in a package until use.

Referring now to FIG. 5, an example method for determining a concentration of a target gas (e.g., target gas 160A) in a sample gas (e.g., sample gas 150) is illustrated. At block 502, example method 500 includes contacting a replaceable testing material (e.g., replaceable testing material 105) with the sample gas (e.g., sample gas 150). In this instance, the replaceable testing material 105 comprises o-tolidine and at least a portion of the sample gas 150 is the target gas 160A. The placement of the replaceable testing material 105 may enable the sample gas 150 containing the target gas 160A to interact with the replaceable testing material 105. For example, contacting the replaceable testing material 105 with the sample gas 150 may include exposing the replaceable testing material 105 to a flow of sample gas 150 (e.g., a sample gas flow). In a non-limiting example, the replaceable testing material 105 may be disposed in a gas detection apparatus and exposing the replaceable testing material 105 to the sample gas flow may include directing a sample gas flow toward the replaceable testing material 105 in the gas detection apparatus.

In some embodiments, determining the concentration of the target gas 160A in the sample gas 150 may optionally include initially determining a concentration of an oxidized derivative 160B of the target gas and correlating the determined concentration of the oxidized derivative 160B to the concentration of the target gas 160A. For example, in an instance in which nitric oxide is the target gas 160A, the sample gas 150 may be subjected to an oxidization chamber 115 and/or exposed to an oxidizing agent 120 in order to transfer an oxygen atom to the nitric oxide molecules, resulting in the oxidized derivative 160B nitrogen oxide (NO₂). Nitrogen dioxide may be more stable and less susceptible to reaction with other chemical molecules than nitric oxide. Accordingly, measuring the concentration of the resulting nitrogen dioxide may provide an easier and a more accurate representation of the amount of nitric oxide in the sample gas 150 than attempting to directly measure the concentration of nitric oxide.

With continued reference to FIG. 5, at block 504, example method 500 includes identifying a color of the replaceable testing material 105. For example, in some embodiments wherein the target gas 160A is present in the sample gas 150, the replaceable testing material 105 may be configured to change color and/or color shade based at least in part on the presence of the target gas 160A and/or the oxidized derivative 160B of the target gas, such as described herein with respect to FIG. 4. For example, in some embodiments, the replaceable testing material 105 may turn from a white or off-white color to blue, green, and/or blue-green and/or shades of blue, green, and/or blue-green in the portion(s) of the replaceable testing material 105 contacted by the sample gas flow such that the identified color of the replaceable testing material 105 is blue, green, and/or blue-green. For example, in a non-limiting example, *o*-tolidine present in or on the surface of the replaceable testing material 105 may react with nitrogen dioxide (e.g., the oxidized derivative 160B of nitric oxide) present in the sample gas 150 to form nitroso-*o*-tolidine. In doing so, the portion(s) of the replaceable testing material 105 contacted by the sample gas flow may initially turn to yellowish-orange. In an instance wherein such replaceable testing material 105 is further exposed to, for example, air, the color may subsequently change to one or more shades of blue, green, and/or blue-green for a period of time (during which the concentration of the target gas 160A and/or oxidized derivative 160B may be determined) due to the presence of moisture (e.g., H₂O) in the air. In some instances, the replaceable testing material 105 may further turn to a darker color, such as brown, gray, and/or black.

In another non-limiting example, wherein water is included as at least a portion of the solvent, *o*-tolidine and water present in or on the surface of the replaceable testing material 105 may react with nitrogen dioxide (e.g., the oxidized derivative 160B of nitric oxide) present in the sample gas 150 to form nitro-*o*-tolidine. In doing so, the portion(s) of the replaceable testing material 105 contacted by the sample gas flow may initially turn to one or more shades of blue, green, and/or blue-green for a period of time at which time the concentration may be determined. In some instances, the replaceable testing material 105 may further turn to a darker color, such as brown, gray, and/or black upon exposure to air.

Although it is possible to manually and/or visually determine the color and/or color shade of the replaceable testing material 105, in some embodiments, a controller 210 may utilize various hardware and software (e.g., image processing circuitry 306) to determine the color and/or shade of the replaceable testing material 105. In some embodiments, a controller (e.g., controller 210) receives from an imaging device (e.g., imaging device 215), an image of the replaceable testing material (e.g., replaceable testing material 105). In some embodiments, captured imagery data may be received at regular intervals by the controller 210, for example, based on the frame rate of the imaging device 215. In some embodiments, imagery data may be received upon request of the controller 210. The imagery data may be transmitted in any format, for example, image format (e.g., JPEG, PNG, GIF, BMP, TIFF, etc.) and/or video format (e.g., MP4, AVI, MKV, MOV, WMV, etc.). Similarly, the imagery data may be transmitted utilizing any transmission standard (e.g., H.264, MPEG-4, G.265, HEVC, VP8, VP9, etc.). The processor 301 may utilize various hardware and/or software components, for example, imaging device interface circuitry 305 to receive and decode the received imagery data. In some embodiments, the controller 210 may utilize image processing algorithms and techniques to identify the replaceable testing material 105 and determine the color and/or color shade of the replaceable testing material 105.

As further illustrated in FIG. 5, at block 506, example method 500 includes determining the concentration of the target gas 160A in the sample gas 150 based at least in part on the identified color of the replaceable testing material 105. For example, in some embodiments, a color and/or shade of the replaceable testing material 105 as identified at block 504 may change based on the concentration of the target gas 160A and/or the oxidized derivative 160B of the target gas such that the color and/or shade may be correlated with the concentration of the target gas 160A and/or the oxidized derivative 160B of the target gas. In some embodiments, a controller (e.g., controller 210) may determine the concentration of the target gas 160A and/or the oxidized derivative 160B of the target gas based at least in part on the color and/or shade of the replaceable testing material 105. That is, in some embodiments, the color and/or color shade of the replaceable testing material 105 may represent the concentration of the target gas 160A and/or the oxidized derivative 160B of the target gas. For example, the replaceable testing material 105 may turn blue, green, and/or blue-green in the presence of the target gas 160A and/or the oxidized derivative 160B of the target gas, however, a light shade of blue, green, and/or blue-green may represent a concentration below 1%, a slightly darker shade of blue, green, and/or blue-green may represent a concentration between 1% and 2% and so on. In another non-limiting example, red may represent a concentration below 1%, orange may represent a concentration between 1% and 2%, yellow may represent a concentration between 2% and 4%, and so on. The color and/or shading of a correlated concentration may be determined previous to operation and may be input to the controller 210 as a configuration or operating parameter. In addition, the color and/or shading of a correlated concentration may be learned, for example, through training utilizing known target gases, oxidized derivatives, and/or user input.

In some embodiments, the controller 210 may optionally consider additional parameters when determining the concentration of the target gas 160A and/or the oxidized derivative 160B of the target gas. For example, physical characteristics of the testing environment and/or the replaceable testing material 105 may affect the determined concentration(s). Physical characteristics may include the temperature, humidity, and/or pressure of the testing environment, the size and position of the replaceable testing material 105, and so on. Additional parameters may also include a gas flow rate of the sample gas 150. In some embodiments, the molecular make-up of the target gas 160A, the oxidized derivative 160B of the target gas, and/or the sample gas 150 may also be considered in determining the concentration of the target gas 160A and/or the oxidized derivative 160B of the target gas within the sample gas 150.

Thus, particular embodiments of the subject matter have been described. While this specification contains many specific implementation details, these should not be construed as limitations on the scope of any inventions or of what may be claimed, but rather as description of features specific to particular embodiments of the disclosure. Other embodiments are within the scope of the following claims. Certain features that are described herein in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

Similarly, while operations or steps are depicted in the drawings in a particular order, this should not be understood as requiring that such operations or steps may be performed in the particular order shown or in sequential order, or that all illustrated operations or steps be performed, to achieve desirable results, unless described otherwise. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products. Any operational step shown in broken lines in one or more flow diagrams illustrated herein are optional for purposes of the depicted embodiment.

In some cases, the actions recited in the claims can be performed in a different order and still achieve desirable results. In addition, the processes depicted in the accompanying figures do not necessarily require the particular order shown, or sequential order, to achieve desirable results, unless described otherwise. In certain implementations, multitasking and parallel processing may be advantageous.

### Overview of Terms

The following explanations of terms are provided to better describe the present disclosure and to guide those of ordinary skill in the art in the practice of the present disclosure.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

As used herein, the phrases "in one embodiment," "according to one embodiment," "in some embodiments," and the like generally refer to the fact that the particular feature, structure, or characteristic following the phrase may be included in at least one embodiment of the present disclosure. Thus, the particular feature, structure, or characteristic may be included in more than one embodiment of the present disclosure such that these phrases do not necessarily refer to the same embodiment.

As used herein, the terms "illustrative," "example," "exemplary" and the like are used to mean "serving as an example, instance, or illustration" with no indication of quality level. Any implementation described herein as "exemplary" or "example" is not necessarily to be construed as preferred or advantageous over other implementations.

The terms "about," "approximately," "generally," "substantially," or the like, when used with a number, may mean that specific number, or alternatively, a range in proximity to the specific number, as understood by persons of skill in the art field and may be used to refer to within manufacturing and/or engineering design tolerances for the corresponding materials and/or elements as would be understood by the person of ordinary skill in the art, unless otherwise indicated.

If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

If the specification presents a list, unless stated otherwise, it is to be understood that each individual element of that list, and every combination of components of that list, is a separate embodiment. For example, "1, 2, 3, 4, and 5" encompasses, among numerous embodiments, 1; 2; 3; 1 and 2; 3 and 5; 1, 3, and 5; and 1, 2, 4, and 5.

The term "plurality" refers to two or more items.

The term "set" refers to a collection of one or more items.

The term "or" is used herein in both the alternative and conjunctive sense, unless otherwise indicated.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described herein. The materials, methods, and examples are illustrative only and not intended to be limiting, unless otherwise indicated. Other features of the disclosure are apparent from the detailed description and the claims.

### Conclusion

Many modifications and other embodiments of the present disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

While the present disclosure has been particularly described in conjunction with specific examples, it is evident that many alternatives, modifications, and variations will be apparent to those skilled in the art in light of the foregoing description. It is therefore contemplated that the appended claims will embrace any such alternatives, modifications, and variations. That is, while various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. The disclosed embodiments relate primarily to the detection of nitric oxide and/or its oxidized derivative, nitrogen dioxide, in a sample gas 150, however, one skilled in the art may recognize that such principles may be applied to any target gas 160A in any environment. For example, other gases and/or volatile organic compounds (VOCs) may also be detected using these techniques. Some example gases may include but are not limited to hydrogen sulfide (H₂S), ammonia (NH₃), carbon monoxide (CO), formaldehyde (HCHO), benzene (C₆H₆), and other similar gases. Alternative embodiments that result from combining, integrating, and/or omitting features of the embodiment(s) are also within the scope of the disclosure. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

## Claims

1. A method for determining a concentration of a target gas in a sample gas, the method comprising:
contacting a replaceable testing material with the sample gas, wherein at least a portion of the sample gas is the target gas and wherein the replaceable testing material comprises o-tolidine;
identifying a color of the replaceable testing material; and
determining the concentration of the target gas in the sample gas based at least in part on the identified color of the replaceable testing material.

2. The method of Claim 1, wherein determining the concentration of the target gas in the sample gas comprises:
determining a concentration of an oxidized derivative of the target gas; and
correlating the determined concentration of the oxidized derivative to the concentration of the target gas.

3. The method of Claim 2, wherein the target gas is nitric oxide and the oxidized derivative is nitrogen dioxide.

4. The method of Claim 1, wherein contacting the replaceable testing material with the sample gas comprises exposing the replaceable testing material to a sample gas flow.

5. The method of Claim 4, wherein the replaceable testing material is disposed in a gas detection apparatus and exposing the replaceable testing material to the sample gas flow comprises directing a sample gas flow toward the replaceable testing material in the gas detection apparatus.

6. The method of Claim 5, wherein the sample gas comprises the target gas and after contacting the replaceable testing material with the sample gas, the replaceable testing material changes color based at least in part on a presence of the target gas.

7. The method of Claim 6, wherein the identified color of the replaceable testing material is blue, green, or a combination thereof.

8. The method of Claim 1, wherein the replaceable testing material is prepared by:
preparing a solution comprising a solute and a solvent, wherein the solute comprises o-tolidine;
introducing a testing material substrate to the prepared solution for a period of time; and
removing at least a portion of the solvent from the testing material substrate such that the testing material substrate comprises at least a portion of the o-tolidine, and thereby forming the replaceable testing material.

9. The method of Claim 8, wherein removing at least the portion of the solvent from the testing material substrate comprises applying absorption paper to the testing material substrate.

10. The method of Claim 9, wherein removing at least the portion of the solvent from the testing material substrate comprises exposing the testing material substrate to a nitrogen gas flow.

11. A reusable gas detection apparatus for determining a concentration of a target gas in a sample gas, the reusable gas detection apparatus comprising:
a replaceable testing material configured to change color based at least in part on a presence of a target gas in the sample gas or an oxidized derivative of the target gas in the sample gas, wherein the replaceable testing material comprises o-tolidine; and
a controller configured to determine the concentration of the target gas.

12. The reusable gas detection apparatus of Claim 11, further comprising:
an imaging device,
wherein the controller is configured to determine the concentration of the target gas based at least in part on correlating the color of the replaceable testing material in one or more images of the replaceable testing material captured by the imaging device to the concentration of the target gas.

13. The reusable gas detection apparatus of Claim 11, wherein the controller is configured to determine the concentration of the target gas based at least in part on determining a concentration of the oxidized derivative of the target gas.

14. The reusable gas detection apparatus of Claim 13, further comprising:
an oxidization chamber comprising an oxidizing agent,
wherein the oxidizing agent oxidizes at least a portion of the target gas in the sample gas to the oxidized derivative of the target gas prior to the sample gas reaching the replaceable testing material.

15. The reusable gas detection apparatus of Claim 14, further comprising:
an integrated display configured to present a result of the determined concentration of the target gas in the sample gas.
